# EUROPÄISCHE PATENTSCHRIFT

(11) **EP 3 638 092 B1**
(45) Veröffentlichungstag und Bekanntmachung des Hinweises auf die Patenterteilung: **02.06.2021**
(21) Anmeldenummer: 18731406.7
(22) Anmeldetag: 12.06.2018
(51) Int. Cl.: A61B 1/00, A61B 17/10, A61B 17/08, A61B 17/128, A61B 1/018, A61B 17/00

(54) **GEWEBECLIP-APPLIKATIONS-AUSRÜST-/NACHRÜSTSATZ**
TISSUE CLIP APPLICATION FITTING/RETROFITTING SET
KIT D'ÉQUIPEMENT/POST-ÉQUIPEMENT POUR APPLICATION COMME CLIP POUR TISSUS

(30) Priorität: 12.06.2017 DE 102017112896
(43) Veröffentlichungstag der Anmeldung: 22.04.2020
(73) Patentinhaber: Ovesco Endoscopy AG, 72074 Tübingen (DE)
(72) Erfinder: SCHURR, Marc, 72072 Tübingen (DE); GOTTWALD, Thomas, 82431 Kochel am See (DE); ANHÖCK, Gunnar, 72764 Reutlingen (DE); BAUR, Franziska, 72622 Nürtingen (DE); SCHOSTEK, Sebastian, 72072 Tübingen (DE); HO, Chi-Nghia, 72762 Reutlingen (DE)
(74) Vertreter: Winter, Brandl - Partnerschaft mbB
(86) Internationale Anmeldenummer: PCT/EP2018/065493
(87) Internationale Veröffentlichungsnummer: WO 2018/229047

(56) Entgegenhaltungen:
- EP-A1- 2 316 350
- WO-A1-2012/174431
- WO-A1-2017/070183
- US-A1- 2013 325 039

## Beschreibung

Die vorliegende Erfindung betrifft einen Gewebeclip-Applikations-Ausrüst- oder Nachrüstsatz mit einem Kappenaufsatz für ein medizinisches Endoskop gemäß dem Oberbegriff des Patentanspruchs 1.

### Hintergrund der Erfindung

Endoskope sind allgemein medizinische Arbeitsgerätschaften zur visuellen Exploration und ggf. Manipulation von Hohlräumen in einem Patientenkörper. Sie weisen grundsätzlich optische Einrichtungen am distalen, d.h. patientenzugewandten Endoskopende (auch Endoskopkopf genannt) sowie optional einen oder mehrere Arbeitskanäle auf, der/die sich ausgehend von einem proximalen (patientenabgewandten) Endoskopabschnitt (der gewöhnlich aus dem Patientenhohlraum nach Außen ragt) oder extrakorporalen Endoskopgriff durch einen (daran sich anschließenden) flexiblen oder starren Endoskopschaft hindurch bis zum Endoskopkopf erstreckt und das extrakorporale Einführen und Zuführen eines oder mehrere medizinische Instrumente wie z.B. eine Zange, Anker, Schere, Nadel, Schlinge, Messer und dergleichen, ermöglicht.

Derartige Endoskope können wahlweise mit zusätzlichen Fähigkeiten versehen werden, etwa indem am distalen Endoskopende/Endoskopkopf eine Kappe oder Hülse auf den zumindest die Optik aufnehmenden/beinhaltenden Endoskopkopf radial außenseitig aufgesetzt wird, die mit bestimmten Funktionen/Funktionselementen versehen oder ausgerüstet ist. Dadurch kann das Endoskop nicht nur zur ursprünglich bestimmungsgemäßen Exploration und/oder als Zugang für minimalinvasive (medizinische) Instrumente sondern als ein solches (minimalinvasives) Instrument selbst zur Ausführung eines interventionellen oder chirurgischen Vorgangs genutzt werden.

### Stand der Technik

Aus dem Stand der Technik beispielsweise der Anmelderin selbst (z.B. DE 20 2008 007 774 U1) ist ein solcher Kappenaufsatz bekannt, der wahlweise auf den Kopf (distaler Endabschnitt, in welchem zumindest eine Optik platziert ist) eines ggf. allgemein bekannten Endoskops der Schaftbauart radial außenseitig aufgesetzt/aufgestülpt werden kann und der an einem radialen (Außen-)umfang einen Gewebeclip in aufgespanntem Zustand trägt, der mittels des Endoskops in den Körperhohlraum eines Patienten eingeführt werden kann. Radial außerhalb des Endoskopschafts oder innerhalb eines im Endoskopschaft ausgebildeten Arbeitskanals (falls vorhanden) ist eine Clip-Abziehvorrichtung, beispielsweise ein Zugfaden geführt (es kann auch eine Druckleitung, ein Schiebedraht oder dergleichen Kraftübertragungsmittel sein), die mit dem Gewebeclip direkt oder indirekt, beispielsweise über einen Schieber oder Kolben zusammenwirkt und von Außerhalb des Körperhohlraums, vorzugsweise am Griff des Endoskops betätigt werden kann, um den Gewebeclip vom Kappenaufsatz in Richtung distal abzuziehen.

Diese Technik erlaubt ein Aus-/Nachrüsten eines (Standard-) Endoskops, um dieses zu einem Gewebeclip-Applizier-Instrument umzufunktionieren.

Aus Platzgründen und aufgrund der hygienischen Notwendigkeit, einen solchen Kappenaufsatz für ein Endoskop als Einweg-Produkt auszubilden, hat sich der Zugfaden oder der Schiebedraht/Druckstab als Kraftübertragungsmittel für ein Abziehen des Gewebeclips gegenüber anderen, wahlweise verwendbaren Betätigungsmitteln (hydraulisch, pneumatisch, elektromotorisch) durchgesetzt. Er ist preisgünstig, einfach zu führen und zu handhaben, sowie zuverlässig für ein Abziehen/Auslösen eines an einer Mantelfläche des Kappenaufsatzes in gespanntem Zustand gelagerten Gewebeclips. In der Regel wird beispielsweise der Zugfaden durch den Arbeitskanal innerhalb des Endoskopschafts oder durch einen außenseitig am Endoskopschaft montierten/montierbaren Zusatzkanal hindurch bis zum proximalen Endoskopendabschnitt geführt, durchgreift am distalen Ende des Endoskops eine von der distalen Vorderkante des Kappenaufsatzes beabstandete Radialbohrung in Richtung nach Außen und wird dann zum Gewebeclip oder zu einem unmittelbar hinter dem Gewebeclip gelagerten Schieber bzw. Abziehring in Richtung proximal zurückgeführt.

An dieser Stelle sei darauf hingewiesen, dass der (bekannte) Kappenaufsatz zumindest in seinem distalen Endabschnitt innenseitig hohlförmig ausgestaltet ist, sodass zu klammerndes Gewebe beispielsweise durch Aufbau von Unterdruck (Ansaugdruck) oder mittels eines durch den Arbeitskanal hindurchgeführten Instruments (Fasszange/Haken/Anker) in den Kappenaufsatz (teilweise) gezogen werden kann. Daraufhin braucht nur noch am Faden gezogen zu werden, um den Gewebeclip in Richtung distal zu verschieben und vom Kappenaufsatz abzulösen. Die im Clip gespeicherte Aufspreiz-Energie wird dabei freigegeben und der Clip verklammert den in den Kappenaufsatz eingezogenen Gewebeabschnitt.

Ferner ist aus EP 2 316 350 A1 eine Resektionsvorrichtung mit einer napfförmigen Kappe bekannt, die am distalen Ende eines Einführschafts befestigt ist und die einen Spreizhülsenabschnitt hat, auf den ein federelastisch vorgespannter Gewebeclip aufgesteckt ist, der mittels einer Auslöse- oder Abziehvorrichtung über die distale Stirnkante der Kappe abziehbar ist, wobei im Inneren des Spreizhülsenabschnitts eine Schneidevorrichtung angeordnet ist, die an der Innenwand des Spreizhülsenabschnitts in einem vorbestimmten axialen Abstand zur distalen Stirnkante der Kappe gehalten ist.

Da der Kappenaufsatz (adaptive Aufsetzkappe) aus hygienischen Gründen als Einweg-Produkt konzipiert sein soll, sind Herstellungskosten möglichst niedrig zu halten. Daher sieht der Stand der Technik eine einfache Kappenform mit möglichst wenigen Bearbeitungsschritten für dessen Herstellung und möglichst wenig beweglichen Bauteilen vor, wodurch die Benutzbarkeit des bekannten Kappenaufsatzes ggf. eingeschränkt werden kann.
- Insbesondere hat es sich als schwierig erwiesen, das zu behandelnde Patientengewebe in korrekter und sicherer Weise zu ergreifen und zu manipulieren.
- Als ein weiteres Problem hat sich in der Vergangenheit die in der Regel kreisrunde Querschnittsform des Kappenaufsatzes herausgestellt. Wird nämlich bei einer kreisrunden Querschnittsform des Kappenaufsatzes und insbesondere bei einer kreisrunden Querschnittsform von dessen in Richtung distal offenen inneren Aushöhlung zu klammerndes Patientengewebe in den Kappenaufsatz/dessen Aushöhlung eingesaugt/eingezogen, so wird das eingezogene Patientengewebe im Wesentlichen kreissymmetrisch deformiert (zusammengedrückt). Insbesondere bei einer chirurgischen Inzision mittels Skalpell oder in der Elektrochirurgie ist eine solche kreissymmetrische/kreisförmige Deformation des Patientengewebes um den Gewebeschnitt herum wenig sinnvoll, da in der Regel zu viel an Patientengewebe (längs des Gewebeschnitts) in die Aushöhlung eingezogen werden muss, um den chirurgischen Schnitt in seiner gesamten Schnittlänge zu klammern.

Günstiger wäre an dieser Stelle ggf. eine ovale Kappenform, die jedoch schwieriger herstellbar ist und auch ungünstiger mit einem im Querschnitt kreisrunden Endoskop gekoppelt werden kann. Außerdem eignet sich eine ovale Form weniger für ein problemloses (atraumatisches) Einführen in den Patientenhohlraum.
- Schließlich ist das Applizieren eines an einer umfänglichen Außenseite eines Kappenaufsatzes aufgespannten/gelagerten Gewebeclips insbesondere in engen Patientenhohlräumen schwierig.

Moderne medizinische Endoskope besitzen in der Regel am distalen Endabschnitt ihres jeweiligen Endoskopschafts einen Abkrümmabschnitt. Hierbei handelt es sich um einen biegeflexiblen Schaftabschnitt des Endoskops, der aktiv mittels geeigneter Kraftübertragungsmittel (Bowdenzüge, Hydraulikeinrichtungen, etc.) von Außerhalb des Patientenhohlraums über den aus dem Patienten endseitig herausragenden Endoskopschaft (passiv biegeflexibel oder starr) wahlweise gekrümmt/abgewinkelt werden kann (vergleichbar mit dem Finger einer menschlichen Hand), um beispielsweise den distal sich daran anschließenden Endoskopkopf in Richtung proximal zu wenden. Da der Endoskopkopf mit einer optischen Einrichtung versehen ist kann auf diese Weise die Optik zur Seite oder sogar nach Hinten in Richtung proximal aktiv ausgerichtet werden.

Wird somit der Abkrümmabschnitt aktiv bezüglich der Schaftlängsachse abgekrümmt, kann die distale Stirnseite des Endoskopkopfs beispielsweise einer Hohlraumwand (Darmwand, Speiseröhrenwand, Magenwand, etc.) des Patienten zugewandt werden. Ist jedoch ein Kappenaufsatz gemäß vorstehender Beschreibung zusätzlich auf den Endoskopkopf aufgesetzt, wird dessen axiale Länge quasi in Richtung distal künstlich gestreckt, sodass insbesondere in engen Hohlräumen ein aktives Abkrümmen des schafteigenen Abkrümmabschnitts problematisch werden kann. Insofern wäre es vorteilhaft, den Kappenaufsatz möglichst kurz zu bauen, wobei jedoch darauf zu achten ist, dass die im Kappenaufsatz ausgeformte Aushöhlung noch ausreichend Patientengewebe aufnehmen kann, um ein sicheres Applizieren des Gewebeclips zu gewährleisten.

### Kurzbeschreibung der Erfindung

In Anbetracht vorstehend genannter Probleme ist es die Aufgabe der vorliegenden Erfindung, einen gattungsgemäßen Kappenaufsatz gemäß vorstehender Definition bereit zu stellen, der eine bessere Handhabbarkeit gegenüber dem bekannten Stand der Technik aufweist.

Ein bevorzugtes Ziel der vorliegenden Erfindung ist es, den Kappenaufsatz so zu konstruieren, dass auch chirurgische Inzisionen mit vergleichsweise großer Schnittlänge durch den Gewebeclip geklammert werden können, ohne dass zu viel Patientengewebe in die napfförmige Aushöhlung des Kappenaufsatzes gezogen werden muss.

Ferner ist es ein bevorzugtes Ziel der vorliegenden Erfindung, das Applizieren eines vom Kappenaufsatz getragenen Gewebeclips insgesamt zu erleichtern.

Schließlich ist es ein Ziel der vorliegenden Erfindung, die zur Erreichung der vorstehenden Ziele notwendigen konstruktiven Maßnahmen am gattungsgemäßen Kappenaufsatz in Einklang mit der grundsätzlichen Zielsetzung zu bringen, den Kappenaufsatz samt Clip-Abziehvorrichtung als Einweg-Produkt betriebswirtschaftlich sinnvoll herstellen zu können.

Die Aufgabe sowie die weiteren optionalen Ziele wird/werden durch einen gattungsgemäßen Gewebeclip-Applikations-Ausrüst- oder Nachrüstsatz mit einem (adaptiven) Kappenaufsatz (Aufsatzkappe) mit den Merkmalen des Patentanspruchs 1 gelöst. Vorteilhafte Ausgestaltungen der Erfindung sind Gegenstand der Unteransprüche.

Ein Kern oder Aspekt der vorliegenden Erfindung besteht demzufolge darin, den (adaptiven, als Nachrüstsatz konzipierten) Kappenaufsatz vorzugsweise gemäß der vorstehenden Definition, ausgebildet und vorgesehen zum (adaptiven) Aufsetzen/Aufstülpen auf den radialen Außenumfang eines (eine Optik aufweisenden) Endoskopkopfs eines medizinischen Endoskops der Schaftbauart, an seiner distalen umlaufenden Vorderkante, die sich im distalen Übergang von der inneren Aushöhlung zu einer in Richtung nach Außen ausgerichteten Umfangs-/Mantelfläche des Kappenaufsatzes ausbildet, zumindest an einer Winkelposition einzukerben (unter Ausbildung zumindest einer in Achsrichtung/längs sich in den Kappenaufsatz einschneidenden Kerbe/Schlitz/Nut), deren Querschnitt beispielsweise V- oder U-förmig sein kann. Die Einkerbung oder auch axial verlaufende Ausnehmung kann sich zudem über einen bestimmten Teilumfang der distalen Stirnkante beispielsweise etwa ein Viertel oder ein Drittel des Gesamtumfangs der distalen Stirnkante erstrecken.

Die dadurch entstehende (in Axialrichtung ausgenommene) Kerbe/Ausnehmung/Spalt kann scharfkantig und/oder abgerundete Kanten aufweisen. Auch sei darauf hingewiesen, dass die vorstehend erwähnte in Richtung nach Außen ausgerichtete Umfangs-/Mantelfläche des Kappenaufsatzes, auf welcher der Clip aufsitzt, nicht die radial äußerste Mantelfläche des (im Wesentlichen zylindrisch gestalteten) Kappenaufsatzes sein muss, welche mit dem Patientengewebe in Anlage kommt sondern beispielsweise durch einen Ringspalt in der Wandung des Kappenaufsatzes gebildet werden kann oder von einer weiteren zylindrischen Wand oder Abdeckung zumindest abschnittsweise sowie umfangsseitig umgriffen werden kann. In diesen letztgenannten Fällen würde der Gewebeclip in den Ringspalt von distal in Richtung proximal eingesetzt und von dem radial äußersten Wandteil radial nach Außen überdeckt/überlagert sein.

Ferner sind in der vorliegenden Erfindung gemäß dem vorstehenden Aspekt wenigstens zwei Einkerbungen oder wenigstens zwei in Axialrichtung sich erstreckende Ausnehmungen an der distalen Vorderkante des Kappenaufsatzes ausgebildet, die bevorzugt dem Aufbau der vorstehend genannten Kerbe/Ausnehmung entsprechen. Die zwei Kerben oder axial sich erstreckende Ausnehmungen sind in einem bestimmten Winkelabstand zueinander, vorzugsweise diametral gegenüberliegend angeordnet. Auch können mehr als zwei Kerben/axial sich erstreckende Ausnehmungen an der distalen Stirnkante des Kappenaufsatzes vorgesehen sein, die sämtlich die (im Wesentlichen) gleiche Querschnittsform aufweisen, oder unterschiedlich zueinander ausgeformt sein können. Auch können die Kerbtiefen (in Axialrichtung gesehen) gleich oder unterschiedlich zueinander sein. Schließlich kann der Kerbgrund parallel oder schief zur distalen Stirnkante verlaufen-

Unabhängig davon, ob wie viele Kerben vorgesehen sind, bewirken die zumindest zwei Kerben ein von einem Kreisrund abweichendes, beispielsweise ovales oder sicherförmiges Einziehen des zu klammernden Patientengewebes in die innere Aushöhlung des (zylindrischen) Kappenaufsatzes auch dann, wenn die distale Stirnkante kreisrund ausgeformt ist (etwa weil der Kappenaufsatz zylinderförmig ist).

In anderen Worten ausgedrückt, ist zu beobachten, dass dann, wenn der Kappenaufsatz an dessen distaler Stirnseite an ein Patientengewebe (flächig/kreisförmig) aufgesetzt und das vom Kappenaufsatz (räumlich) umfasste Gewebe per Unterdruck oder mechanisch (z.B. durch eine Fasszange) in die innere Aushöhlung des Kappenaufsatzes eingezogen wird, dieses durch die zumindest eine Kerbe (beim Einziehen) beispielsweise ovalfömig (d.h. von Kreisrund abweichend) deformiert wird. Auf diese Weise kann beispielsweise das eine Inzision (Längsschnitt) umgebende Gewebe bei minimalem Gewebeüberschuss (Längs des Schnittes) in die Aushöhlung eingezogen und sicher geklammert werden.

Zusammenfassend wird die der Erfindung zugrunde liegende Aufgabe durch einen Gewebeclip-Applikations-Ausrüst-oder Nachrüstsatz mit einem Kappenaufsatz gelöst, der dafür ausgebildet ist, auf den distalen Kopf eines medizinischen Endoskops der Schaftbauart aufgestülpt zu werden und der hierfür einen proximalen Aufstülpabschnitt und einen distalen Gewebeclip-Halteabschnitt aufweist, in dessen Bereich innenseitig ein in Richtung distal offener Hohlraum ausgebildet ist, der am distalen Ende des Kappenaufsatzes durch eine umlaufende Vorderkante begrenzt ist, die sich im distalen Übergang vom Hohlraum zu einer in Richtung nach außen ausgerichteten Umfangsfläche des Kappenaufsatzes ausbildet, auf der im Bereich des Gewebeclip-Halteabschnitts einen Gewebeclip radial abgestützt ist, wobei die umlaufende Vorderkante zumindest zwei in Axialrichtung sich erstreckende Einkerbungen, Nuten, Ausnehmungen oder Rücksprünge aufweist, die in einem bestimmten Winkelabstand zueinander, vorzugsweise diametral an der Vorderkante ausgebildet oder angeordnet sind, wobei ein Kappenaufsatz-eigenen Arbeitskanal bereitgestellt ist, der in den Hohlraum mündet und in einem Bereich distal zum Aufstülpabschnitt sowie proximal zum radial abgestützten Gewebeclip radial aus dem Hohlraum tritt, um dann separat zu dem Endoskop längsgeführt werden zu können, ein erstes Führungs-oder Leitelement, das in dem Hohlraum angeordnet ist, sich in Axialrichtung erstreckt sowie in Richtung distal gesehen radial in den Hohlraum neigt und das dem Kappenaufsatz -eigenen Arbeitskanal in Richtung distal nachgeordnet ist, derart, dass ein in den Kappenaufsatz -eigenen Arbeitskanal eingeschobenes erstes medizinisches Instrument am ersten Führungs-oder Leitelement radial in Richtung Hohlraum abgelenkt wird ein zweites Führungs-oder Leitelement, das in einem Winkelabstandvorzugsweise diametral zum ersten Führungs-oder Leitelement innerhalb des Hohlraums angeordnet ist und sich in Axialrichtung erstreckt sowie in Richtung distal gesehen radial in den Hohlraum neigt, welches dafür vorgesehen ist, sich in Verlängerung zu einem Endoskop-eigenen Arbeitskanal auszurichten, derart, dass ein in den Endoskop-eigenen Arbeitskanal zusätzlich zu dem ersten medizinischen Instrument eingeschobenes zweites medizinisches Instrument am zweiten Führungs-oder Leitelement radial in Richtung Hohlraum abgelenkt wird.

Gemäß einem weiter bevorzugten, in Kombination mit dem vorstehenden Aspekt und ggf. dessen Weiterbildungen zu beanspruchenden Aspekt der vorliegenden Erfindung ist der Kappenaufsatz nicht gänzlich zylinderförmig (bzw. gerade) sondern in einem Mittelabschnitt (unter Ausbildung eines Winkels, vorzugsweise kleiner 90° beispielsweise zwischen 5° bis 15° zur Aufsatz-Längsachse in Aufsatzlängsrichtung) gekröpft bzw. abgebogen, derart, dass zumindest die distale Vorderkante des Kappenaufsatzes (gemäß vorstehender Definition), vorzugsweise jener Axialabschnitt, in welchem die innere Aushöhlung ausgebildet ist und weiter vorzugsweise jener Axialabschnitt, auf welchem der Gewebeclip aufgespannt/aufgelagert ist, zur Aufsatzlängsachse abgewinkelt ist (wobei in diesem Fall die distale Stirnkante in einer zum abgebogenen Abschnitt senkrechten Ebene liegt). Um somit einen Gewebeclip zu applizieren, muss der Endoskop-seitige aktive Abkrümmabschnitt, der sich proximal zum Endoskopkopf und damit zum Kappenaufsatz anordnet, deutlich weniger aktiv abgekrümmt werden, um die distale Vorder-/Stirnkante des Kappenaufsatzes der zu behandelnden Wand des Patientenhohlraums zuzuwenden.

Gemäß einem weiteren Aspekt der vorliegenden Erfindung ist innerhalb des Kappenaufsatzes ein Längskanal angeordnet, der bevorzugt mit dem Kappenaufsatz einstückig ausgebildet ist oder der fest mit dem Kappenaufsatz verbunden ist und seitlich, d.h. an der Mantelseite des Kappenaufsatzes proximal zum Gewebeclip oder dessen Abziehrichtung radial nach Außen tritt, um dann längs des Endoskopschafts an dessen Außenseite zu verlaufen (vorzugsweise durch Clips oder Bänder am Endoskopschaft temporär gehalten). Über diesen Längskanal kann beispielsweise ein chirurgisches Instrument wie eine Fasszange in die innere Aushöhlung vorgeschoben werden oder es kann über dem Längskanal ein Unterdruck (gegenüber Atmosphäre) in dem inneren Hohlraum (innere Aushöhlung) erzeugt werden.

Dadurch wird es möglich, sowohl durch den zumindest einen Arbeitskanal des (Standard-) Endoskops als auch über den zusätzlichen weiteren Arbeitskanal des Kappenaufsatzes (separat zum Endoskop-Arbeitskanal) jeweils ein medizinisches Instrument beispielsweise zum Ergreifen oder Halten von Patientengewebe in den inneren Hohlraum des Kappenaufsatzes einzuführen und so das zu behandelnde Patientengewebe zeitgleich an zwei beabstandeten Greifpunkten in den inneren Hohlraum einzuziehen. Vorzugsweise öffnet sich der zusätzliche, Kappenaufsatz-eigene Arbeitskanal im Bereich der / in Längsrichtung zur zumindest einen Kerbe, sodass das Patientengewebe sicher über die zumindest eine Kerbe bzw. in Axialrichtung sich erstreckende Ausnehmung(en) in den inneren Hohlraum des Kappenaufsatzes eingezogen werden kann.

Gemäß einem weiteren Aspekt der vorliegenden Erfindung ist der erfindungsgemäße Kappenaufsatz mit einem vorzugsweise rampenförmigen Abweiser oder Ablenkelement versehen, der vorzugsweise einstückig im inneren Hohlraum des Kappenaufsatzes ausgeformt ist und sich in Axialrichtung erstreckt. Dabei ist der Abweiser bzw. das Ablenkelement in Richtung distal gesehen zusätzlich radial nach innen ausgerichtet. Er kann ferne eine Art Rinne oder Kanal bilden in der beispielsweise ein medizinisches Instrument längsgeführt werden kann.

Wird der Kappenaufsatz auf das distale Ende eines Endoskops (d.h. auf den Endoskopkopf) aufgestülpt, kann der Kappenaufsatz so auf dem Endoskopkopf gedreht werden, bis sich der Abweiser bzw. das Ablenkelement zur Öffnung des Endoskopinternen Arbeitskanals in Axialrichtung ausrichtet (quasi eine Verlängerung des Endoskop-eigenen Arbeitskanals bildet). Wird somit ein medizinisches Instrument durch den Endoskop-eigenen Arbeitskanal in den inneren Hohlraum des Kappenaufsatzes vorgeschoben, erfährt der distale Endabschnitt des medizinischen Instruments durch den Kappen-eigenen Abweiser eine (geringfügige) geführte Ablenkung zur Hohlraummitte. Dadurch kann das Patientengewebe zentraler ergriffen und dadurch besser und sicherer in den inneren Hohlraum der Aufsatzkappe gezogen werden.

Vorzugsweise ist der kappen-interne, zusätzliche Arbeitskanal ebenfalls in Axialrichtung gesehen zur Hohlraummitte hin geneigt.

Nach einem weiteren Aspekt der Erfindung ist distal zur Öffnung des kappen-internen, zusätzlichen Arbeitskanal ein weiterer Abweiser bzw. Ablenkelement mit vorstehendem Aufbau und Funktion ausgebildet/angeordnet.

Des Weiteren kann (insbesondere im Fall eines gekröpften Kappenaufsatzes) ein zusätzliches optisches Element wie z.B. ein Spiegel an der Innenwand des Kappenaufsatzes angeordnet oder ausgebildet sein, um die Sicht ausgehend von Endoskopkopf bzw. dessen Optik in Richtung hin zur distalen Vorderkante des Aufsatzes zu verlängern und/oder der Kappenaufsatz ist zumindest abschnittsweise aus einem transparenten Material, um die Sicht in Richtung distal auch durch die Abwinkelung nicht oder nur leicht zu behindern. Schließlich ist es aber auch möglich, den Kappenaufsatz mit einer eigenen Optik (separat/zusätzlich zur standardgemäß vorhandenen Endoskopoptik) auszurüsten. Grundsätzlich kann zumindest die Beleuchtung des Endoskops auch weiterhin verwendet werden, wobei es natürlich auch denkbar ist, den Kappenaufsatz mit einer eigenen Lichtquelle, zusätzlich zur Endoskopinternen Beleuchtung, auszurüsten, wodurch der erfindungsgemäße Kappenaufsatz unabhängig von der Ausstattung des (Standard-)Endoskops autark eingesetzt werden kann.

An dieser Stelle sei darauf hingewiesen, dass aus dem Stand der Technik insbesondere auch gemäß der Anmelderin Applizier-Instrumente bekannt sind (weisen keine Optiken auf und stellen damit auch keine Endoskope gemäß allgemeiner Definition dar), deren (starrer) Instrumentenschaft unmittelbar vor dem Instrumentenkopf (distaler Instrumenten-Endabschnitt) einfach oder S-förmig gekröpft/gebogen ist, um die distale Stirnseite des Instrumentenkopfs der Wand eines Patientenhohlraums zuzuwenden. Im vorliegenden Fall aber wird/ist nicht der Endoskopkopf bzw. der Schaft unmittelbar vor (proximal zu) dem Endoskopkopf gekröpft (was mittels des aktiv betätigbaren Abkrümmabschnitts ermöglicht werden könnte). Vielmehr kann der den Clip tragende Kappenaufsatz, der dem Endoskopkopf und dessen darin aufgenommene Optik axial (in Richtung distal) nachgelagert ist, bevorzugt gekröpft sein. Insbesondere erfolgt die Abkrümmung des Kappenaufsatzes in einem axialen Bereich, der von einem Montage- /Aufsteckabschnitt (elastische Muffe) des Kappenaufsatzes in Richtung distal beabstandet ist.

Auf diese Weise muss der Endoskopschaft im Bereich des dem Endoskopkopf und damit dem Kappenaufsatz unmittelbar vorgeschalteten Abwinklungsabschnitt nur noch geringfügig (oder überhaupt nicht) aktiv abgewinkelt werden, um ein exaktes Applizieren des Gewebeclips zu ermöglichen.

Abschließend sei nochmals explizit darauf hingewiesen, dass die vorstehend genannten Aspekte (sowie deren vorteilhafte Weiterbildungen) beliebig miteinander bedarfsweise kombinierbar sind.

So können beispielsweise die zumindest eine Frontkerbe oder mehrere umfangsbeabstandete Kerben nicht nur bei dem herkömmlichen zylindrischen (gerade sich erstreckenden) Kappenaufsatz vorgesehen sein, sondern auch bei dem gekröpften Kappenaufsatz. Auch ist die Form der zumindest einen Kerbe nicht auf eine bestimmte Form beschränkt sondern kann beliebig ausgestaltet sein wie etwa rechteckig, dreieckig, trapezförmig, teilkreisförmig, etc. Es kann auch eine Zacken- oder Wellenform an der Vorderkante des Kappenaufsatzes vorgesehen sein.

Die Abkröpfung kann in einem Winkelbereich kleiner 90°, vorzugsweise zwischen 5° und 15° liegen.

Der Kappenaufsatz ist dafür ausgebildet, an einer Mantelseite von diesem einen Gewebeclip zu lagern. Zusätzlich kann der Kappenaufsatz aber auch mit einer eigenen Gewebetrennvorrichtung beispielsweise in Form eines Lasers oder einer elektrischen Schneideschlinge bestückt sein, die an der Innenfläche der inneren Aushöhlung gehalten/geführt ist und über eine im Endoskopschaft vorzugsweise durch dessen Arbeitskanal geführt Leiterlitze bestromt/betätigt werden kann.

Schließlich kann der Kappenaufsatz zusätzlich mit einer Art Überwurfhülse ausgebildet sein, die Fest mit dem Kappenaufsatz (vorzugsweise stoffeinstückig) verbunden ist und den auf die eine Mantelfläche aufgezogenen Gewebeclip radial (zumindest abschnittsweise) umgibt.

Vorzugsweise kann die in Richtung nach Außen ausgerichtete Umfangs-/Mantelfläche des Kappenaufsatzes (auf welcher der Gewebeclip unmittelbar gelagert ist) mit einer Längsnut ausgebildet sein, die sich in axialer Verlängerung zu einer, einen Ziehfaden führenden Radialbohrung erstreckt und zur Axial-/Längsführung des aus der Radialbohrung austretenden Ziehfadens an der in Richtung nach Außen weisenden Umfangs-/Mantelfläche vorgesehen ist. Auf diese Weise wird der Ziehfaden im Bereich der Mantelfläche des Kappenaufsatzes quasi in die Kappenwand versenkt und kann somit vom Gewebeclip bei dessen Abziehbewegung ungehindert überfahren werden.

Wie aus der vorstehenden Kurzbeschreibung zu entnehmen ist, ist es für den Erfolg der vorliegenden Erfindung, nämlich die gezielte Deformation des in den Kappenaufsatz eingezogenen Gewebes für ein optimiertes Applizieren des Gewebeclips entscheidend, dass der Kappenaufsatz an seinem distalen Kappenrand wenigstens zwei, bevorzugt diametral sich gegenüberliegende Einkerbungen oder (Längs-)Schlitze aufweist, wobei der Kappenaufsatz mit einem eigenen Arbeitskanal ausgebildet ist, der vorzugsweise an einem Umfangsrandbereich des Kappenaufsatzes in den vom Kappenaufsatz gebildeten Hohlraum zur Aufnahme des eingezogenen Gewebes mündet. Darüber hinaus ist sowohl der Mündungsstelle des Kappenaufsatz-eigenen Arbeitskanals nachgeordnet, wie auch an einer bevorzugt diametral gegenüberliegenden Stelle in Überstimmung mit den beiden Einkerbungen ein Abweiser oder eine Rampe (Keil) ausgebildet, der dafür ausgebildet und vorgesehen ist, ein durch den Kappenaufsatz-eigenen Arbeitskanals eingeführtes medizinisches Greifinstrument wie auch ein durch den Arbeitskanal des Endoskops gleichzeitig eingeführtes medizinisches Greifinstrument radial nach Innen abzulenken, sodass sich die beiden gleichzeitig eingeführten medizinischen Greifinstrumente sich nicht gegenseitig behindern.

Durch die Kombination dieser Maßnahmen wird die beabsichtige Deformation des durch die Einkerbungen hindurchgezogenen, von den Greifinstrumenten in unmittelbarer Nähe zu den Einkerbungen erfassten Gewebes erreicht.

### Kurzbeschreibung der Figuren

Die Erfindung wird nachstehend anhand bevorzugter Ausführungsbeispiele unter Bezugnahme auf die begleitenden Figuren näher erläutert.
Fig. 1 zeigt einen Kappenaufsatz gemäß einem ersten bevorzugten Ausführungsbeispiel der vorliegenden Erfindung in Vorbereitung zur Montage auf einem Endoskopkopf eines vorzugsweise allgemein bekannten Endoskops,
Fig. 2 zeigt einen distalen Endabschnitt des erfindungsgemäßen Kappenaufsatzes nach Fig. 1 mit hierzu alternativer Stirnkantenform und
Fig. 3 zeigt einen Kappenaufsatz gemäß einem zweiten bevorzugten Ausführungsbeispiel der vorliegenden Erfindung in Vorbereitung zur Montage auf den Endoskopkopf des vorzugsweise allgemein bekannten Endoskops nach Fig. 1.
Fig. 4 zeigt zwei beispielhafte Anwendungsfälle a) und b) eines Gewebeclip-Applikations-Ausrüst- oder Nachrüstsatzes gemäß dem ersten bevorzugten Ausführungsbeispiel.

### Figurenbeschreibung

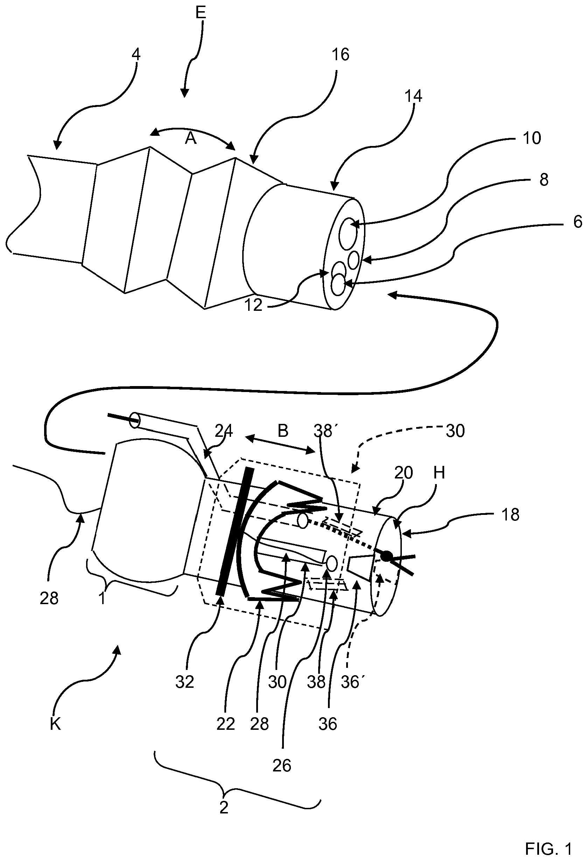

Der in der Fig. 1 schematisch dargestellte Gewebeclip-Applikations-Ausrüst- oder Nachrüstsatz hat einen oder besteht aus einem Kappenaufsatz K, der im Wesentlichen einen proximalen Aufstülpabschnitt 1 und einen distalen Gewebeclip-Halteabschnitt 2 aufweist.

Der Aufstülpabschnitt 1 ist in Form einer Muffe oder Manschette aus einem flexiblen, vorzugsweise elastischen Material gebildet. Alternativ ist es aber auch möglich, den Aufstülpabschnitt 1 aus einer Kunststoffhülse zu fertigen, die radial aufweitbar ist (z.B. durch Anordnung wenigstens eines Längsschlitzes oder infolge einer vorbestimmten Eigenflexibilität). Der Aufstülpabschnitt 1 ist dafür vorbereitet, auf das distale Ende eines (handelsüblichen) Endoskops E vorzugsweise bekannter Bauart an dessen Außenseite aufgestülpt zu werden.

In der Regel hat ein solches Endoskop E einen (biegeflexiblen oder starren) Endoskopschaft 4, an dessen distalem Ende zumindest eine Optik 6 und eine Beleuchtungseinrichtung 8 eingebaut sind. Des Weiteren hat ein Endoskop E bekannter Bauart häufig einen internen Arbeitskanal 10, über den ein medizinisches Instrument beispielsweis in ein Patienten-Hohlorgan einführbar ist. Schließlich kann am distalen Ende des Endoskops E noch eine Spülvorrichtung 12 vorgesehen sein, mittels der die Optik 6 gereinigt werden kann. Im Nachfolgenden wird jener distale Endabschnitt des Endoskops E, in welchem zumindest die Optik 6 untergebracht ist, als Endoskopkopf 14 bezeichnet. Diesem Endoskopkopf 14 kann optional ein Endoskopschaftabschnitt proximal vorgeordnet sein, der aktiv von einem Bediener abgekrümmt werden kann, um die Optik 6 zur Seite oder ggf. sogar in Richtung proximal auszurichten. Dieser Abschnitt wird nachfolgend als Abkrümmabschnitt 16 des Endoskops E bezeichnet.

Der Aufstülpabschnitt 1 des erfindungsgemäßen Kappenaufsatzes K ist nunmehr dafür vorgesehen, auf den Endoskopkopf 14 gemäß vorstehender Definition aufgestülpt zu werden (nicht in den Endoskop-Arbeitskanal 6 eingeführt zu werden), um so quasi eine axiale Verlängerung des Endoskopkopfs 14 bzw. des distalen Endabschnitts des Endoskopschafts 4 zu bilden. Der Endoskop-eigene Arbeitskanal 6 bleibt somit für das Einführen eines medizinischen Instruments offen.

Der Gewebeclip-Halteabschnitt 2 besteht aus einer gegenüber dem Aufstülpabschnitt 1 vorzugsweise starreren (hohlen) Kunststoffhülse, die in Verlängerung zum Aufstülpabschnitt 1 einstückig, vorzugsweise stoffeinstückig mit diesem verbunden oder mit dem Aufstülpabschnitt 1 beispielsweise durch Verkleben oder Verschweißen gekoppelt ist. Der Gewebeclip-Halteabschnitt 2 bildet innenseitig eine Aushöhlung oder einen Hohlraum H, der zur temporären Aufnahme von Patientengewebe vorgesehen ist und der am distalen Ende des Gewebeclip-Halteabschnitts 2 unter Ausbildung einer distalen Vorder- oder Stirnkante 18 in eine radial äußere Mantelfläche 20 übergeht, auf der ein Gewebeclip 22 (in aufgespanntem Zustand) axialbeweglich aufgelagert ist.

Innerhalb des Kappenaufsatzes K ist ein Arbeitskanal 24 angeordnet oder ausgebildet, der mit dem Kappenaufsatz K (stoff-) einstückig ausgeformt oder mit dem Kappenaufsatz fest verbunden ist sowie in die innere Aushöhlung/Hohlraum H in Richtung distal mündet. Der Kappenaufsatz-eigene Arbeitskanal 24 (separat zum Endoskop-eigenen Arbeitskanal 10) ist bevorzugt im Bereich des Aufstülpabschnitts 1 oder distal davon radial zur Außenseite des Kappenaufsatzes K geführt, um sich dann über eine vorbestimmte Länge wenigstens entsprechend der zu erwartenden Länge des Endoskopschafts 1 (ca. 2m) zu erstrecken, derart, dass ein extrakorporales Einführen eines minimalinvasiven medizinischen Instruments in den Arbeitskanal 24 des Kappenaufsatzes K (separat zum in der Regel vorhandenen Arbeitskanal des Endoskops) möglich ist.

In einem distalen Endbereich des Kappenaufsatzes K ist eine Radialbohrung 26 ausgebildet, welche den inneren Hohlraum H mit der mantelseitigen Umgebung verbindet und durch welchen ein Abziehfaden 28 geführt ist. An der den Gewebeclip 22 radial stützenden Mantel-/Umfangsfläche des Gewebeclip-Halteabschnitts 2 ist gemäß der Fig. 1 ferner eine in Richtung außen offene Axialnut 30 ausgeformt, die in axialer Richtung auf die Radialbohrung 28 zuläuft. Schließlich ist auf der den Gewebeclip 22 radial stützenden Mantel-/Umfangsfläche des Gewebeclip-Halteabschnitts 2 eine Abziehvorrichtung vorliegend mit einem Abzieh-/Gleitring 32 gelagert, der unmittelbar proximal zum Gewebeclip 22 positioniert ist und an welchen der Abziehfaden 28 für ein extrakorporal ausgeführtes vorzugsweise manuelles Bewegen des Gleitrings 32 in Richtung distal gekoppelt ist.

Der Abziehfaden 28 ist hierfür durch die Axialnut 30 zunächst in Richtung distal in die Radialbohrung 26 geführt, innerhalb der Radialbohrung 26 umgelenkt und schließlich beispielsweise durch den Arbeitskanal 24 des Kappenaufsatzes K oder den Arbeitskanal 10 des Endoskops E oder durch einen weiteren separaten Kanal in Richtung proximal zum extrakorporalen proximalen Ende des Endoskops E zurückgeführt.

Wie schließlich in der Fig. 1 durch Strichlinien angedeutet ist, kann die den Gewebeclip lagernde Umfangsfläche des Kappenaufsatzes K zusätzlich von einer Hülse oder axial sich ersteckende Zunge 34 radial überlagert sein, die den Gewebeclip 22 radial nach Außen zumindest abschnittsweise überdeckt.

Gemäß dem ersten bevorzugten Ausführungsbeispiel der vorliegenden Erfindung weist die distale Stirn- oder Vorderkante 18 zumindest eine erste Einkerbung bzw. einen ersten, in axialer Richtung sich erstreckenden Rücksprung 36 auf. Wie die Fig. 1 offenbart, können auch zwei Einkerbungen/Rücksprünge 36, 36' an der distalen Vorderkante 18 des Gewebeclip-Halteabschnitts 2 ausgebildet sein, die sich bevorzugt diametral gegenüberliegen. Sie können aber auch in jedem beliebig anderen Winkelabstand zueinander positioniert sein. Des Weiteren weisen die beiden Einkerbungen bevorzugt die gleiche Form und Dimensionierung auf. Auch können mehr als zwei Kerben vorgesehen sein.

Abschließend sei noch darauf hingewiesen, dass der Kappenaufsatz K in dessen Hohlraum H wenigstens eine Art Führungselement (Rampe) 38 aufweist, das sich in Axialrichtung sowie in Richtung distal gesehen radial nach innen erstreckt. Das Führungs- oder Leitelement 38 ist mit einer Längsnut oder Rinne (gestrichelt dargestellt) versehen, in der beispielsweise ein medizinisches Instrument längsgeführt werden kann. Bei richtiger Montage des erfindungsgemäßen Kappenaufsatzes K auf dem Endoskopkopf bildet das Führungselement 38 eine axiale Verlängerung zum Arbeitskanal 10 des Endoskops E.

Der Kappenaufsatz-interne Arbeitskanal 24 kann ebenfalls in Richtung distal gesehen radial nach innen geneigt sein und/oder es ist ein weiteres Führungselement (Rampe) 38' dem Kappenaufsatz-eigenen Arbeitskanal 24 distal nachgeschaltet, das sich in Axialrichtung erstreckt und gleichzeitig radial nach innen in den Hohlraum geneigt ist. Schließlich kann jedes Führungselement 38, 38' mit einer Längsnut oder Rinne ausgebildet sind.

Die Funktion des Gewebeclip-Applikations-Ausrüst- oder Nachrüstsatzes gemäß der Fig. 1 lässt sich wie Folgt beschreiben:
Zunächst wird der erfindungsgemäße Kappenaufsatz K auf den Kopf 14 des (allgemein bekannten) Endoskops E an dessen äußerer Umfangsseite aufgesetzt. Für den richtigen Sitz in Axialrichtung kann zwischen dem Aufstülpabschnitt 1 und dem Gewebeclip-Halteabschnitt 2 ein innerer Axialanschlag, z. B. ein radial nach innen gerichteter umlaufender Ringvorsprung oder Zapfen (nicht weiter gezeigt) vorgesehen sein, der sich auf die distale Stirnseite des Endoskopkopfs 14 anlegt. Ferner wird der Kappenaufsatz K auf dem Endoskopkopf 14 solange gedreht, bis sich das zumindest eine Führungselement 38 in Axialrichtung zur Austrittsöffnung des Endoskop-eigenen Arbeitskanals 10 ausrichtet.

Der Aufsatz-eigene Arbeitskanal 24 ist in dem vorliegenden Beispiel im Bereich des Aufstülpabschnitts 14 oder unmittelbar distal zu diesem radial nach Außen aus dem Hohlraum H des Aufsatzes K herausgeführt und erstreckt sich so außerhalb entlang des vorzugsweise flexiblen Endoskopschafts 4, 16 des (allgemein bekannten) Endoskops E in Richtung proximal nach Außerhalb des zu behandelnden Patientenhohlorgans.

Wie in der Fig. 1 zu sehen ist, hat das Endoskop E zwischen Kopf 14 und Schaft 4 den vorstehend genannten Abwinkelabschnitt 16 (auch Abwinklungseinheit genannt), der aktiv von Außerhalb des Patientenhohlraums/Patientenhohlorgans wahlweise abgekrümmt (in Richtung Pfeil A) werden kann. Auf diese Weise kann der Endoskopkopf 14 samt darauf aufgestülptem Kappenaufsatz K zu einer Hohlorganwand weisend ausgerichtet werden.

Sobald der Kappenaufsatz K distal stirnseitig, d.h. mit seiner distalen Vorderkante 18 (die ggf. abgerundet ist) gegen das Gewebe der Hohlorganwand des Patienten angepresst ist, kann das Patientengewebe in die innere Aushöhlung des Kappenaufsatzes K eingezogen werden. Dies erfolgt mittels in der Aushöhlung aufgebauten sowie beispielsweise über den Aufsatz-eigenen Arbeitskanal 24 erzeugten Unterdruck und/oder mittels eines über den Aufsatz-eigenen Arbeitskanal 24 eingeführten Instruments 24 (Zange, Haken, Gewebeanker, etc.). Zusätzlich kann über den Endoskop-eigenen Arbeitskanal 10 ein weiteres medizinisches Instrument in den Hohlraum H des Kappenaufsatzes K eingeführt werden, um so das zu behandelnde Patientengewebe an zwei voneinander beabstandeten Stellen zu erfassen und in den kappenseitigen Hohlraum H ziehen.

Beim Einführen des/der medizinischen Instrument(e) in den Hohlraum H gleiten diese an den Rampen 38, 38'bzw. in deren Längsnuten ab und werden so nach radial innen in den Hohlraum H gedrückt. Auf diese Weise kann das Patientengewebe besser gegriffen werden. Während des Einziehens des Patientengewebes in den Hohlraum H wird dieses auch durch die zumindest eine Kerbe 36, 36' gezogen und dabei deformiert. Es hat sich dabei gezeigt, dass insbesondere bei Anordnung mindestens einer oder besser zweier Einkerbungen 36, 36' im Bereich der distalen Vorderkante 18 des Kappenaufsatzes K, welche beispielsweise diametral zueinander positioniert sind, das Patientengewebe nicht mehr kreissymmetrisch sondern eher oval in die Aushöhlung H eingezogen wird. Somit kann eine ausreichende Gewebemasse beispielsweise längs eines Gewebeschnitts problemlos in die Aufsatzkappe eingezogen werden.

Sobald ausreichend Gewebe in die Aushöhlung H eingezogen ist, wird der Gewebeclip 22 mittels des Ziehfadens 28 in Richtung distal über die Vorderkante 18 abgestreift (längs Pfeil B), wobei dieser hierauf das Patientengewebe zusammenklemmt. Dabei über fährt der Gewebeclip 18 fortlaufend den Ziehfaden 28, ohne diesen aber zu beeinflussen, das dieser in der Axialnut 30 versenkt ist.

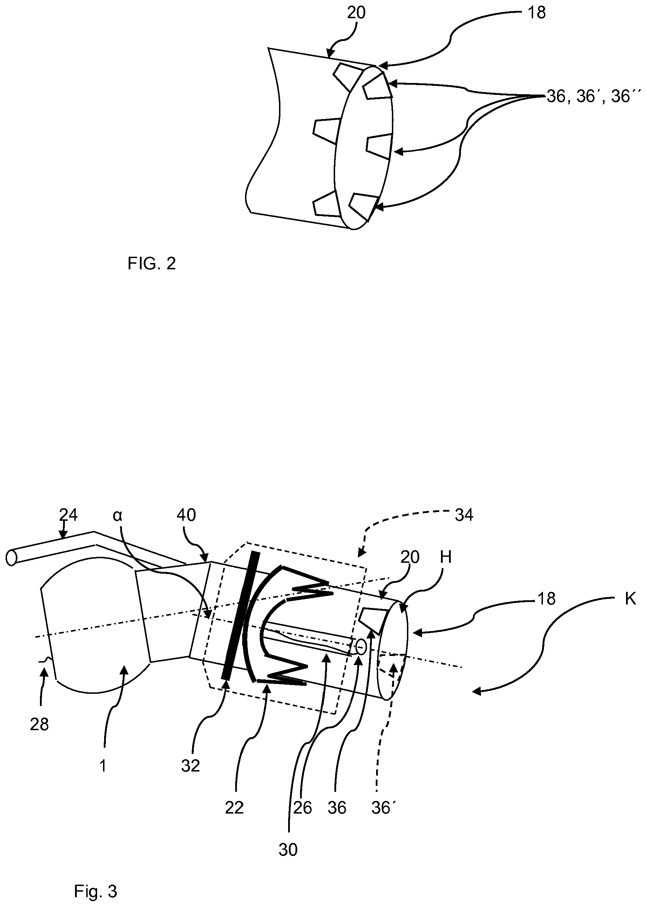

In der Fig. 2 ist eine Abwandlung zu dem Ausführungsbeispiel gemäß der Fig. 1 dargestellt.

In diesem Fall sind mehr als zwei Einkerbungen 36 an der distalen Vorderkante 18 des erfindungsgemäßen Kappenaufsatzes K ausgeformt, von denen zumindest zwei Einkerbungen beispielsweise im Wesentlichen diametral zueinander positioniert sind (nicht unbedingt erforderlich). Sämtliche weiteren Merkmale dieser Abwandlung entsprechen jenen des ersten Ausführungsbeispiels gemäß der Fig. 1.

Die Fig. 3 zeigt ein zweites Ausführungsbeispiel der vorliegenden Erfindung. Im Nachfolgenden werden nur jene Merkmale detailliert beschrieben, in denen sich das zweite Ausführungsbeispiel vom ersten Ausführungsbeispiel der vorliegenden Erfindung unterscheidet. Dabei sei darauf hingewiesen, dass der Kappenaufsatz K des zweiten Ausführungsbeispiels ebenfalls mit distalen Einkerbungen 36 an der Vorderkante 18 des Kappenaufsatzes sowie mit einer Längsnut 30 gemäß vorstehender Beschreibung ausgebildet sein kann, wobei diese Merkmale beim zweiten Ausführungsbeispiel als rein optionale Merkmale zu verstehen sind. Außerdem können auch Führungselemente vergleichbar zum ersten bevorzugten Ausführungsbeispiel vorgesehen sein.

Im Unterschied zum ersten Ausführungsbeispiel ist der Kappenaufsatz K des zweiten Ausführungsbeispiels nicht gerade-zylindrisch sondern gekröpft ausgebildet. In anderen Worten ausgedrückt, weist der Kappenaufsatz in einem Mittelabschnitt, der vorzugsweise den Aufstülpabschnitt 1 vom Clip-Halteabschnitt 2 separiert, eine Abknickstelle/-kante 40 auf, an welcher der Kappenaufsatz K in einem Winkel zur Aufsatz-Längsachse abgelenkt/abgewinkelt ist, d.h. in einem Winkel α vorzugsweise kleiner als 90°, weiter vorzugsweise zwischen 5°bis 15° von der Aufsatz-Längsachse abweicht. Die Abkröpfstelle 40 liegt beispielsweise proximal zur inneren Aushöhlung H, d.h. beispielsweise proximal zum Abstreif-/Gleitring 32.

Um in diesem Fall die Funktion des Endoskops E, auf dessen Kopf 14 der Kappenaufsatz K gemäß Fig. 3 aufgestülpt ist, noch zu gewährleisten, ist der Abkröpfwinkel α bevorzugt so gewählt, dass das Sichtfeld/Blickrichtung der Endoskop-eigenen Optik 6 sowie dessen Beleuchtung 8 die Öffnung der Aufsatz-eigenen Aushöhlung gerade noch erfassen kann. Außerdem werden Endoskop-interne Funktionen wie z.B. Reinigung der Endoskop-Optik nicht beeinflusst. Alternativ oder zusätzlich ist der Kappenaufsatz K aus einem durchsichtigen Material gefertigt und/oder es sind Einrichtungen (nicht gezeigt) innerhalb des Kappenaufsatzes K vorgesehen, die das Blickfeld/Blickrichtung der Endoskop-eigenen Optik und ggf. dessen Beleuchtung in Richtung Öffnung der Aushöhlung umlenken/weiterleiten. Alternativ oder zusätzlich kann der Aufsatz K aber auch mit einer eigenen Optik (nicht dargestellt) versehen sein.

Die vorliegende Erfindung betrifft zusammenfassend einen Gewebeclip-Applikations-Ausrüst- oder Nachrüstsatz mit einem Kappenaufsatz, der dafür ausgebildet ist, auf den distalen Kopf eines medizinischen Endoskops der Schaftbauart aufgestülpt zu werden und der hierfür einen proximalen Aufstülpabschnitt und einen distalen Gewebeclip- Halteabschnitt aufweist. Erfindungsgemäß kann der Kappenaufsatz in einem den Aufstülpabschnitt vom Halteabschnitt trennenden Mittenabschnitt vorzugsweise unter Ausbildung eines Winkels α zur Aufsatz-Längsachse größer 0°gekröpft sein. Ferner kann die distale Vorderkante des Kappenaufsatzes zumindest an einer Winkelposition unter Ausbildung zumindest einer in Axialrichtung sich in den Kappenaufsatz einschneidenden Kerbe/Schlitz/Nut 28 eingekerbt sein.

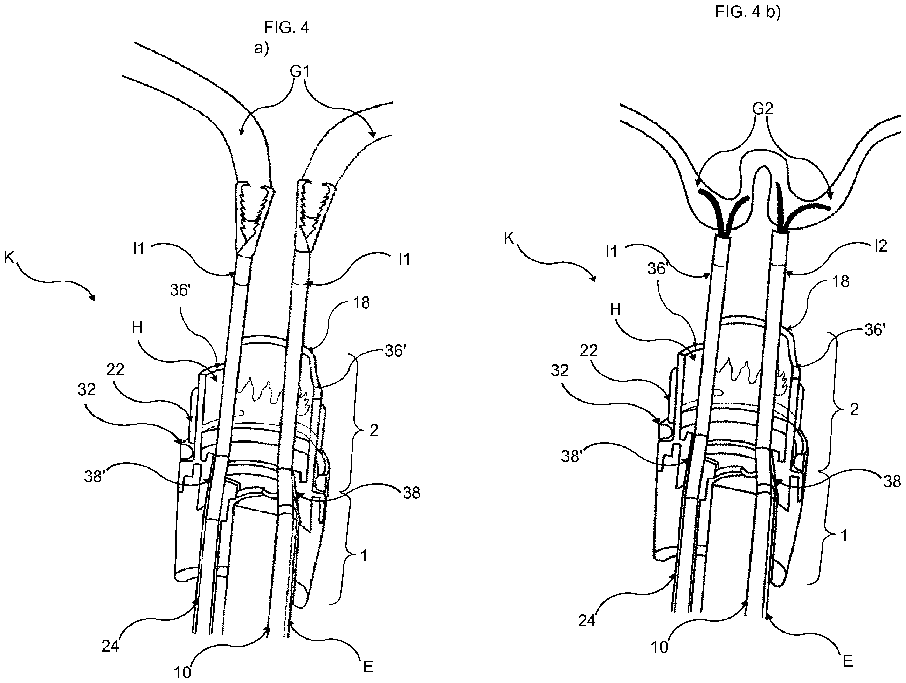

Fig. 4 zeigt beispielhaft zwei Anwendungsfälle eines Gewebeclip-Applikations-Ausrüst-oder Nachrüstsatzes gemäß dem ersten bevorzugten Ausführungsbeispiel, um eine der der Erfindung zugrunde liegenden Problematiken zu veranschaulichen. Diese besteht darin, zwei voneinander getrennte Gewebeschichten G1 oder Gewebefalten G2 sicher und unter minimaler Gewebebeschädigung zu greifen und zu klammern, wobei die Gewebefalten G2 mithilfe des Gewebeclip-Applikations-Ausrüst- oder Nachrüstsatzes erst erzeugt werden müssen. Dies ist insbesondere durch die im Stand der Technik gezeigten Systeme nicht oder nur unzureichend möglich.

Fig. 4 a) (links) zeigt eine Anwendung des erfindungsgemäßen Gewebeclip-Applikations-Ausrüst- oder Nachrüstsatzes beim Behandeln einer Anastomoseninsuffizienz bzw. einer Gewebeperforation. Dabei müssen zwei oder mehr voneinander getrennte Gewebeschichten G1 separat gegriffen werden, um diese anschließend miteinander zu verbinden/ zu adaptieren, indem sie in den Hohlraum H des Kappenaufsatzes K eingezogen werden, dabei gefaltet werden und anschließend der Gewebeclip 2 darüber abgestreift wird, um sie aneinander zu klammern.

Um die Gewebeschichten G1 separat greifen zu können, ist es notwendig, zwei separate chirurgische Instrumente I1, I2 (z.B. Greifer oder Saugröhren) gleichzeitig durch den Hohlraum H gerichtet zum Patientengewebe vorzuschieben. Hierfür werden zwingend zwei Arbeitskanäle 10, 24 benötigt, um die zwei chirurgischen Instrumente I1, I2 separat voneinander einschieben und betätigen zu können. Ferner müssen beide chirurgischen Instrumente I1, I2 mithilfe der Führungselemente/Rampen 38, 38' aufgerichtete Weise radial nach innen abgelenkt werden, um jeweils gerichtet zu den Gewebeschichten G1 vorgeschoben zu werden, wo die Instrumente I1, I2 die einzelnen Gewebeschichten G1 an zueinander beabstandeten Punkten präzise greifen können. Anschließend werden die Gewebeschichten G1 durch ein Zurückziehen der Instrumente I1, I2 in den Hohlraum H des Kappenaufsatzes K eingezogen. Da die Arbeitskanäle 10, 24, die Führungselemente/Rampen 38, 38' und die Einkerbungen/Rücksprünge 36, 36' in distaler Richtung axial zueinander ausgerichtet sind bzw. in einer durch die Längsachse des Gewebeclip-Halteabschnitts 2 verlaufenden Ebene liegen, legen sich dabei die einzelnen Gewebeschichten G1 in die Einkerbungen/Rücksprünge 36, 36', wodurch das Gewebe gefaltet wird. Abschließend wird der Gewebeclip 22 von dem Gewebecliphalteabschnitt 2 abgestreift und dadurch das Gewebe verbunden/geklammert.

Fig. 4 b) (rechts) zeigt eine Anwendung des erfindungsgemäßen Gewebeclip-Applikations-Ausrüst- oder Nachrüstsatzes bei einer Verengung eines Hohlorgans, beispielsweise einer Magenverkleinerung. Hierfür müssen zwei separate Gewebefalten G2 erst erzeugt werden. Das Vorgehen hierbei entspricht im Wesentlichen dem der vorstehend anhand von Fig. 4 a) beschriebenen Gewebebehandlung. Zur Erzeugung der zwei separaten Gewebefalten G2 ist insbesondere das gerichtete Vorschieben der Instrumente I1, I2 und somit das Ablenken der Instrumente über die Führungselemente/Rampen 38, 38' wichtig, um zwei voneinander beabstandete Gewebepunkte greifen zu können, welche weit genug voneinander entfernt sind, dass sich zwei separate Gewebefalten G2 bilden, wenn die Instrumente I1, I2 das Gewebe greifen und in den Hohlraum H einziehen. Dabei wird das Gewebe über die Einkerbungen/Rücksprünge 36, 36' gefaltet. Die so erzeugten Gewebefalten G2 werden übereinandergelegt und durch Abstreifen des Gewebeclips 22 miteinander geklammert. Auf diese Weise kann eine relativ große Gewebemenge verkleinert werden und somit das Hohlorgan durch nur wenige Klammervorgänge deutlich verengt werden.

Würde das Gewebe nur an einem einzigen Gewebepunkt bzw. an zwei sehr nahe beieinander liegenden Gewebepunkten gegriffen werden, wäre es nicht möglich, zwei separate Gewebefalten G2 zu erzeugen und zu klammern oder zwei voneinander getrennte Gewebeschichten G1 miteinander zu klammern.

## Patentansprüche

1. Gewebeclip-Applikations-Ausrüst- oder Nachrüstsatz mit einem Kappenaufsatz (K), der dafür ausgebildet ist, auf den distalen Kopf eines medizinischen Endoskops (E) der Schaftbauart aufgestülpt zu werden und der hierfür einen proximalen Aufstülpabschnitt (1) und einen distalen Gewebeclip-Halteabschnitt (2) aufweist, in dessen Bereich innenseitig ein in Richtung distal offener Hohlraum (H) ausgebildet ist, der am distalen Ende des Kappenaufsatzes (K) durch eine umlaufende Vorderkante (18) begrenzt ist, die sich im distalen Übergang vom Hohlraum (H) zu einer in Richtung nach außen ausgerichteten Umfangsfläche (20) des Kappenaufsatzes (K) ausbildet, auf der im Bereich des Gewebeclip-Halteabschnitts (2) einen Gewebeclip (22) radial abgestützt ist, wobei die umlaufende Vorderkante (18) zumindest zwei in Axialrichtung sich erstreckende Einkerbungen, Nuten, Ausnehmungen oder Rücksprünge (36, 36') aufweist, die in einem bestimmten Winkelabstand zueinander an der Vorderkante (18) ausgebildet oder angeordnet sind
**gekennzeichnet, durch**
einen Kappenaufsatz-eigenen Arbeitskanal (24), der in den Hohlraum (H) mündet und in einem Bereich distal zum Aufstülpabschnitt (1) sowie proximal zum radial abgestützten Gewebeclip (22) radial aus dem Hohlraum (H) tritt, um dann separat zu dem Endoskop (E) längsgeführt werden zu können,
ein erstes Führungs- oder Leitelement (38'), das in dem Hohlraum (H) angeordnet ist, sich in Axialrichtung erstreckt sowie in Richtung distal gesehen radial in den Hohlraum (H) neigt und das dem Kappenaufsatz -eigenen Arbeitskanal (24) in Richtung distal nachgeordnet ist, derart, dass ein in den Kappenaufsatz -eigenen Arbeitskanal (24) eingeschobenes erstes medizinisches Instrument am ersten Führungs- oder Leitelement (38') radial in Richtung Hohlraum (H) abgelenkt wird
ein zweites Führungs- oder Leitelement (38), das in einem Winkelabstand zum ersten Führungs- oder Leitelement (38') innerhalb des Hohlraums (H) angeordnet ist und sich in Axialrichtung erstreckt sowie in Richtung distal gesehen radial in den Hohlraum (H) neigt, welches dafür vorgesehen ist, sich in Verlängerung zu einem Endoskop-eigenen Arbeitskanal (10) auszurichten, derart, dass ein in den Endoskop-eigenen Arbeitskanal (10) zusätzlich zu dem ersten medizinischen Instrument eingeschobenes zweites medizinisches Instrument am zweiten Führungs-oder Leitelement (38) radial in Richtung Hohlraum (H) abgelenkt wird.

2. Gewebeclip-Applikations-Ausrüst- oder Nachrüstsatz gemäß einem der vorstehenden Ansprüche, **dadurch gekennzeichnet, dass** sich der zusätzliche, Kappenaufsatz-eigener Arbeitskanal (24) im Bereich der / in Längsrichtung zu einer der Einkerbungen, Nuten, Ausnehmungen oder Rücksprünge (36, 36') öffnet.

3. Gewebeclip-Applikations-Ausrüst- oder Nachrüstsatz gemäß Anspruch 1 oder 2, **dadurch gekennzeichnet, dass** die/der zumindest zwei Einkerbungen, Nuten, Ausnehmungen oder Rücksprünge (36, 36') U- oder V-förmig ausgebildet sind.

4. Gewebeclip-Applikations-Ausrüst- oder Nachrüstsatz gemäß einem der vorstehenden Ansprüche, **dadurch gekennzeichnet, dass** der Kappenaufsatz (K) in einem den Aufstülpabschnitt (1) vom Gewebeclip-Halteabschnitt (2) trennenden Mittelabschnitt unter Ausbildung eines Winkels (α) größer 0° und kleiner 90° zu einer Aufsatz-Längsachse gekröpft oder abgekrümmt ist, derart, dass zumindest die distale Vorderkante (18) des Kappenaufsatzes (K), vorzugsweise jener Axialbereich, in welchem der Hohlraum (H) ausgebildet ist und weiter vorzugsweise jener Teil des Gewebeclip-Halteabschnitts (2), auf welchem der Gewebeclip (22) radial abgestützt ist, zur Aufsatz-Längsachse abgewinkelt ist.

5. Gewebeclip-Applikations-Ausrüst- oder Nachrüstsatz gemäß Anspruch 4, **dadurch gekennzeichnet, dass** die umlaufende Vorderkante (18) in einer Ebene liegt, die sich senkrecht zur Längsachse des Teils des Gewebeclip-Halteabschnitts (2), auf welchem der Gewebeclip (22) radial abgestützt ist, aufspannt.

6. Gewebeclip-Applikations-Ausrüst- oder Nachrüstsatz nach einem der vorstehenden Ansprüche, **dadurch gekennzeichnet, dass** die Umfangsfläche (20) des Kappenaufsatzes (K), auf welcher der Gewebeclip (22) unmittelbar radial abgestützt ist, mit einer Längsnut (30) ausgebildet ist, die distal an eine den Hohlraum (H) mit der nach außen ausgerichteten Umfangsfläche (20) verbindenden Radialbohrung (26) im Gewebeclip-Halteabschnitt (2) angrenzt und sich in Axialrichtung hin zum Gewebeclip (22) erstreckt.

7. Gewebeclip-Applikations-Ausrüst- oder Nachrüstsatz nach Anspruch 6, **gekennzeichnet durch** eine Abzieheinrichtung mit einem auf der nach außen gerichteten Umfangsfläche (20) axialgleitend gelagerten Abziehring (32) unmittelbar proximal zum Gewebeclip (22), an den ein Ziehfaden (28) gekoppelt ist, der in der Längsnut (30) zur Radialbohrung (26) geführt ist und diese bis in den Hohlraum (H) durchdringt und der dafür ausgebildet ist, um von dort in Richtung proximal aus einem Patientenhohlraum (H) geführt zu werden.

8. Gewebeclip-Applikations-Ausrüst- oder Nachrüstsatz nach einem der vorstehenden Ansprüche, **gekennzeichnet dadurch, dass** zumindest eines der Führungs- oder Leitelemente (38, 38') eine Rinne oder einen Kanal ausbildet, derart, dass darin entsprechend eines der medizinischen Instrumente längsgeführt werden kann.

## Claims

1. A tissue clip application fitting set or retrofitting set with a cap attachment (K) which is adapted to be put over the distal head of a medical endoscope (E) of the shaft type, and which, for this purpose, has a proximal placement section (1) and a distal tissue clip holding section (2) in the region of which a hollow chamber (H) open in the distal direction is formed internally, said hollow chamber being bordered at the distal end of the cap attachment (K) by a circumferential front edge (18) formed in the distal transition from the hollow chamber (H) to a peripheral surface (20) of the cap attachment (K) which is oriented in an outward direction and on which a tissue clip (22) is supported radially in the region of the tissue clip holding section (2), wherein the circumferential front edge (18) comprises at least two notches, grooves, recesses or offsets (36, 36') extending in the axial direction and formed or arranged on the front edge (18) at a particular angular distance to each other,
**characterized by**
a working channel (24) which is part of the cap attachment and opens into the hollow chamber (H) and exits the hollow chamber (H) radially in a region distal to the placement section (1) and proximal to the radially supported tissue clip (22) to be able to be guided longitudinally separately to the endoscope (E),
a first guiding or leading element (38') which is arranged in the hollow chamber (H), extends in the axial direction and, viewed in the distal direction, inclines radially into the hollow chamber (H), and which is subordinate to the working channel (24) being part of the cap attachment in a distal direction, such that a first medical instrument introduced into the working channel (24) being part of the cap attachement is deflected radially in the direction of the hollow chamber (H) at the first guiding or leading element (38'), and
a second guiding or leading element (38) which is arranged within the hollow chamber (H) at an angular distance to the first guiding or leading element (38') and extends in an axial direction as well as, viewed in the distal direction, inclines radially into the hollow chamber (H), and which is provided to be oriented in extension to a working channel (10) being part of the endoscope, such that a second medical instrument introduced into the working channel (10) being part of the endoscope in addition to the first medical instrument is deflected radially in the direction of the hollow chamber (H) at the second guiding or leading element (38).

2. The tissue clip application fitting set or retrofitting set according to any of the preceding claims, **characterized in that** the additional working channel (24) being part of the cap attachment opens in the region of / in the longitudinal direction to one of the notches, grooves, recesses or offsets (36, 36').

3. The tissue clip application fitting set or retrofitting set according to claim 1 or claim 2, **characterized in that** the at least two notches, grooves, recesses or offsets (36, 36') are of U- or V-shaped design.

4. The tissue clip application fitting set or retrofitting set according to claim any of the preceding claims, **characterized in that** the cap attachment (K) in a central section separating the placement section (1) from the tissue clip holding section (2) is cranked or crooked relative to an attachment longitudinal axis, forming an angle (α) larger than 0° and smaller than 90°, such that at least the distal front edge (18) of the cap attachment (K), preferably the axial region in which the hollow chamber (H) is formed and further preferably the portion of the tissue clip holding section (2) on which the tissue clip (22) is supported radially, is angled relative to the attachment longitudinal axis.

5. The tissue clip application fitting set or retrofitting set according to claim 4, **characterized in that** the circumferential front edge (18) is disposed in a plane which spans perpendicularly to the longitudinal axis of the portion of the tissue clip holding section (2) on which the tissue clip is supported radially.

6. The tissue clip application fitting set or retrofitting set according to any of the preceding claims, **characterized in that** the peripheral surface (20) of the cap attachment (K) on which the tissue clip (22) is directly supported radially, is formed with a longitudinal groove (30) which distally adjoins a radial bore (26) in the tissue clip holding section (2) which connects the hollow chamber (H) with the outwardly oriented peripheral surface (20), and extends in the axial direction toward the tissue clip (22).

7. The tissue clip application fitting set or retrofitting set according to claim 6, **characterized by** a pull-off means including a pull-off ring (32) which is mounted in an axially sliding manner on the peripheral surface (20) directed outwardly and directly proximally to the tissue clip (22), and which has a pulling thread (28) connected thereto, which pulling thread is guided in the longitudinal groove (30) to the radial bore (26) and penetrates the same up to the hollow chamber (H), and is adapted to be guided from there in the proximal direction out of a patient's hollow chamber (H).

8. The tissue clip application fitting set or retrofitting set according to any of the preceding claims, **characterized in that** at least one of the guiding or leading elements (38, 38') forms a chute or a channel such that one of the medical instruments can be correspondingly guided longitudinally therein.

## Revendications

1. Ensemble d'équipement ou de post-équipement pour application de clip pour tissus muni d'un embout formant capuchon (K), qui est conçu pour être emboîté sur la tête distale d'un endoscope médical (E) du type tige et qui comporte à cette fin un segment d'emboîtement proximal (1) et un segment de retenue distal (2) de clip pour tissus, dans la zone duquel est formée du côté interne une cavité (H) ouverte en direction distale, qui est limitée à l'extrémité distale de l'embout formant capuchon (K) par un bord antérieur circulaire (18) qui se développe dans le passage distal de la cavité (H) en une surface circulaire (20) de l'embout formant capuchon (K), orientée en direction de l'extérieur, sur laquelle un clip pour tissus (22) est supporté radialement dans la zone du segment de retenue (2) de clip pour tissus, le bord antérieur circulaire (18) comportant au moins deux rainures, gorges, évidements ou retraits (36, 36') s'étendant en direction axiale, qui sont formé(e)s ou disposé(e)s à une certaine distance angulaire l'un(e) de l'autre sur le bord antérieur (18)
**caractérisé par**
un canal de travail (24) propre à l'embout formant capuchon, qui débouche dans la cavité (H) et sort radialement de la cavité (H) dans une zone en position distale par rapport au segment d'emboîtement (1) ainsi qu'en position proximale par rapport au clip pour tissus (22) supporté radialement, afin de pouvoir ensuite être guidé longitudinalement séparément de l'endoscope (E),
un premier élément de guidage ou de conduite (38') qui est disposé dans la cavité (H), s'étend en direction axiale et, vu en direction distale, s'incline radialement dans la cavité (H) et qui est disposé après, en direction distale, le canal de travail (24) propre à l'embout formant capuchon, de sorte qu'un premier instrument médical inséré dans le canal de travail (24) propre à l'embout formant capuchon est dévié sur le premier élément de guidage ou de conduite (38') radialement en direction de la cavité (H),
un deuxième élément de guidage ou de conduite (38), qui est disposé à une distance angulaire du premier élément de guidage ou de conduite (38') à l'intérieur de la cavité (H) et s'étend en direction axiale et, vu en direction distale, s'incline radialement dans la cavité (H), qui est prévu pour s'aligner en prolongement d'un canal de travail (10) propre à l'endoscope, de sorte qu'un deuxième instrument médical inséré en plus du premier instrument médical dans le canal de travail (10) propre à l'endoscope est dévié sur le deuxième élément de guidage ou de conduite (38) radialement en direction de la cavité (H).

2. Ensemble d'équipement ou de post-équipement pour application de clip pour tissus selon l'une quelconque des revendications précédentes, **caractérisé en ce que** le canal de travail (24) supplémentaire, propre à l'embout formant capuchon, ouvre dans la zone des / en direction longitudinale vers l'un(e) des rainures, gorges, évidements ou retraits (36, 36').

3. Ensemble d'équipement ou de post-équipement pour application de clip pour tissus selon la revendication 1 ou 2, **caractérisé en ce que** lesdit(e)s au moins deux rainures, gorges, évidements ou retraits (36, 36') sont en forme d'U ou de V.

4. Ensemble d'équipement ou de post-équipement pour application de clip pour tissus selon l'une quelconque des revendications précédentes, **caractérisé en ce que** dans un segment médian séparant le segment d'emboîtement (1) du segment de retenue (2) de clip pour tissus l'embout formant capuchon (K) est courbé ou coudé en angle avec formation d'un angle (α) supérieur à 0° et inférieur à 90° par rapport à un axe longitudinal de l'embout, de sorte qu'au moins le bord antérieur distal (18) de l'embout formant capuchon (K), de préférence la zone axiale dans laquelle la cavité (H) est formée et encore mieux la partie du segment de retenue (2) de clip pour tissus sur laquelle le clip pour tissus (22) est supporté radialement, est incliné(e) par rapport à l'axe longitudinal de l'embout.

5. Ensemble d'équipement ou de post-équipement pour application de clip pour tissus selon la revendication 4, **caractérisé en ce que** le bord antérieur circulaire (18) se trouve dans un plan qui s'étend perpendiculairement à l'axe longitudinal de la partie du segment de retenue (2) de clip pour tissus, sur laquelle le clip pour tissus (22) est supporté radialement.

6. Ensemble d'équipement ou de post-équipement pour application de clip pour tissus selon l'une quelconque des revendications précédentes, **caractérisé en ce que** la surface circulaire (20) de l'embout formant capuchon (K), sur laquelle le clip pour tissus (22) est directement supporté radialement, est configurée avec une rainure longitudinale (30), qui est adjacente, dans le segment de retenue (2) de clip pour tissus, en position distale à une forure radiale (26) reliant la cavité (H) à la surface circulaire (20) orientée vers l'extérieur et s'étend en direction axiale vers le clip pour tissus (22).

7. Ensemble d'équipement ou de post-équipement pour application de clip pour tissus selon la revendication 6, **caractérisé par** un dispositif de tirage comportant un anneau de tirage (32) en position immédiatement proximale par rapport au clip pour tissus (22), placé en pouvant glisser axialement sur la surface circulaire (20) orientée vers l'extérieur, auquel est couplé un fil de tirage (28) qui est conduit dans la rainure longitudinale (30) vers la forure radiale (26) et la parcourt jusque dans la cavité (H) et qui est conçu pour, de là, être conduit en direction proximale hors d'une cavité (H) d'un patient.

8. Ensemble d'équipement ou de post-équipement pour application de clip pour tissus selon l'une quelconque des revendications précédentes, **caractérisé en ce qu'**au moins l'un des éléments de guidage ou de conduite (38, 38') forme une gouttière ou un canal de sorte que l'un des instruments médicaux peut être convenablement conduit longitudinalement dans celui-ci/celle-ci.
